# EUROPEAN PATENT SPECIFICATION

(11) **EP 0 614 652 B1**
(45) Date of publication and mention of the grant of the patent: **18.07.2001**
(21) Application number: 94103683.2
(22) Date of filing: 10.03.1994
(51) Int. Cl.: A61F 13/02

(54) **Sticking material for hemostasis**
Klebeverband zur Blutstillung
Pansement pour l'hémostase

(30) Priority: 10.03.1993 JP 7502893; 20.05.1993 JP 13983393; 04.03.1994 JP 6014494
(43) Date of publication of application: 14.09.1994
(73) Proprietor: NICHIBAN CO. LTD., Tokyo 102 (JP)
(72) Inventor: Ito, Toshio, c/o Nichiban Company Limited, Tokyo (JP); Akutsu, Takayuki, c/o Nichiban Company Limited, Tokyo (JP); Saito, Tsugio, c/o Nichiban Company Limited, Tokyo (JP); Numata, Takayoshi, c/o Nichiban Company Limited, Tokyo (JP); Kusumi, Katsumi, c/o Nichiban Company Limited, Tokyo (JP); Tokumura, Fumio, c/o Nichiban Company Limited, Tokyo (JP); Ohyama, Kunihiro, c/o Nichiban Company Limited, Tokyo (JP)
(74) Representative: May, Hans Ulrich, Dr.

(56) References cited:
- EP-A- 0 067 622
- EP-A- 0 141 693
- CH-A- 676 197
- FR-A- 2 499 416
- PATENT ABSTRACTS OF JAPAN vol. 014, no. 149 (C-0705) 22 March 1990 & JP-A-02 019 152 (NICHIBAN CREATE) 23 January 1990
- PATENT ABSTRACTS OF JAPAN vol. 017, no. 708 (C-1147) 24 December 1993 & JP-A-05 245 173 (NICHIBAN) 24 September 1993

## Description

### BACKGROUND OF THE INVENTION

### (1) Field of the invention

The present invention relates to a sticking material for hemostasis, i.e. a hemostatic adhesive bandage or plaster.

### (2) Background information

EP-A-0 067 622 describes a surgical plaster having a stretchable strip carrying a pad, the pad being sufficiently incompressible to insure that the pad will completely flatten and close a vein against escape of blood after a puncture of the vein or blood sampling. The pad is not compressible more than 25 % of its height by the elastic strip, and has a substantially non absorbing surface. While this surgical plaster is suitable to flatten a vein, it is not satisfactory to provide hemostasis after an arteric puncture.

EP-A-O 141 693 describes a compressing hemostatic plaster comprising a body of such form, dimension and rigidity as to exert a compressive action on the point or region of hemorrhage while allowing free blood circulation, the body serving as support for a pad of a non-adherent hemostatic and/or cicatrising material in form of a powder, gel or tablet. The body (2) is fixed to or incorporated in a band (1) which itself may be chosen from woven or unwoven textile materials and may optionally be provided with an adhesive for application to the skin. While this plaster may be suitable to provide hemostasis of a vein, it is not satisfactory to provide hemostasis after an arteric puncture.

In recent years, therapies and tests involving arteric puncture such as arterio puncture catheter, arteriographs or inter arterial injection, have been widely conducted. The arteric puncture catheter is widely used for the purpose of monitoring the blood pressure of artery during operation, and collecting the blood from the artery to check the conditions of the lungs by the blood conditions. The arterio puncture catheter is also applied to hemodialysis or the like. Further, the arteriography is widely used not only for diagnosis, but also for the procedure of arterial embolism and percutaneous trans-catheter angioplasty.

Heretofore, when the therapies and tests involving arterio puncture are conducted, in many cases, after the therapy or the like, the injection needle, catheter, therapeutic tube or the like is removed from the ductus arteriosus, and the area from which it is removed (the puncture area) is pressed with fingers for at least about 10 minutes.

Then, a gauze is placed over the puncture area and a large pillow-like pad is put thereon, and then fastened to apply pressure to the ductus arteriosus for hemostasis. After the hemostasis is ascertained, the patient is returned to a ward or the like. After such therapies involving arterio puncture, the hemostasis treatments is made by doctors or nurses. However, the doctors or nurses have much work to do after the therapy, and it is desired to improve such hemostasis manner effectively.

For the hemostasis of artery, there is no adequate method at present which performs it readily and effectively due to the high blood pressure of about 100 to 150 mmHg.

### SUMMARY OF THE INVENTION

It is an object of the present invention to make it possible to conduct hemostasis readily and securely when the therapies or tests involving the puncture of ductus arteriosus are carried out and then the injection'needle or the like at the puncture area of artery is removed as mentioned above.

This object is solved by the features of independent claim 1.

According to the present invention, at one surface of a base material made of a stretchable material having a recovery property, an adhesive layer is disposed. On the adhesive layer, there is placed a pressing plate and on this plate a pad with an adequate thickness, having a sufficient hardness and a sufficient elasticity to ensure complete flattening and closing of a vein when applied against a puncture area of said vein, the size of the pad being smaller than that of the pressing plate and such that it is usable to press both the puncture of the arterial blood vessel and the puncture of the skin surface at the same time. The sticking material obtained as above is fixed to the wound area in the vicinity of the puncture area by the base material having an adhesive, and the puncture of the arterial blood vessel is pressed tightly for hemostasis with the pad, the pressing action of the pad being strengthened by the pressing plate, while absorbing the bleeding blood from the puncture of the skin surface in the pad, to securely conduct the hemostasis.

Furthermore, according to the present invention, a pad is placed to cover the puncture area of the arterial blood vessel, a pressing plate larger in size than the pad is placed on the skin surface at the opposite side to the skin surface on which the pad is placed with the arterial blood vessel interposed, and then the pad and the pressing plate are fixed with a base material having an adhesive to surround the arterial blood vessel of the wound area, whereby the pad is pressed against the puncture area for hemostasis.

The other objects and features of the present invention will become apparent to persons of ordinary skill in the art by reference to the following description when read in connection with the accompanying drawings.

### BRIEF DESCRIPTION OF THE DRAWINGS

The embodiments of the invention of Fig.1 to 10 are shown without the pressing plate.

Fig.1 is a perspective view of an embodiment of the sticking material for hemostasis of the present invention.

Fig.2 is a cross-sectional view of the sticking material in Fig.1.

Fig.3 is a cross-sectional view showing the sticking material for hemostasis as shown in Fig.1 in the packed condition.

Fig.4 is an explanatory view showing the sticking material for hemostasis as shown in Fig. 1 in use.

Fig.5 is an explanatory view showing the sticking material for hemostasis as shown in Fig. 1 when used for hemostasis of artery.

Fig.6 is a cross-sectional view of another embodiment of the sticking material for hemostasis of the present invention.

Fig.7 is a cross-sectional view showing a further embodiment of the sticking material for hemostasis of the present invention.

Fig.8 is a cross-sectional view showing still another embodiment of the sticking material for hemostasis of the present invention.

Fig.9 is a plan view showing another modified embodiment of the sticking material for hemostasis of the present invention.

Fig.10 is a plan view of a further modified embodiment of the sticking material for hemostasis of the present invention.

Fig.11 is a perspective view showing another embodiment of the sticking material for hemostasis of the present invention.

Fig.12 is a cross-sectional view of the sticking material as shown in Fig.11.

Fig.13, is an explanatory view showing the sticking material for hemostasis as shown in Fig. 11 in use.

Fig.14 is an explanatory view showing the sticking material for hemostasis of the present invention in another use.

Fig.15 is a plan view showing another embodiment of the sticking material for hemostasis of the present invention.

Fig.16 is a plan view showing a further embodiment of the sticking material for hemostasis of the present invention.

Fig.17 is a perspective view showing another embodiment of the sticking material for hemostasis of the present invention.

Fig.18 is a plan view showing still another embodiment of the sticking material for hemostasis of the present invention.

Fig.19 is a plan view showing a further embodiment of the sticking material for hemostasis of the present invention.

Fig.20 is a perspective view showing another embodiment of the sticking material for hemostasis of the present invention.

Fig.21 is a perspective view showing still another embodiment of the sticking material for hemostasis of the present invention.

Fig.22 is a plan view showing another embodiment of the sticking material for hemostasis of the present invention.

Fig.23 is a plan view showing still another embodiment of the sticking material for hemostasis of the present invention.

Fig.24 is a plan view showing the sticking material for hemostasis of the present invention when the substrate thereof is extended.

Fig.25 is an explanatory view showing an apparatus for measuring the pressing force of the sticking material for hemostasis of the present invention.

### DETAILED DESCRIPTION OF THE PREFERRED EMBODIMENTS

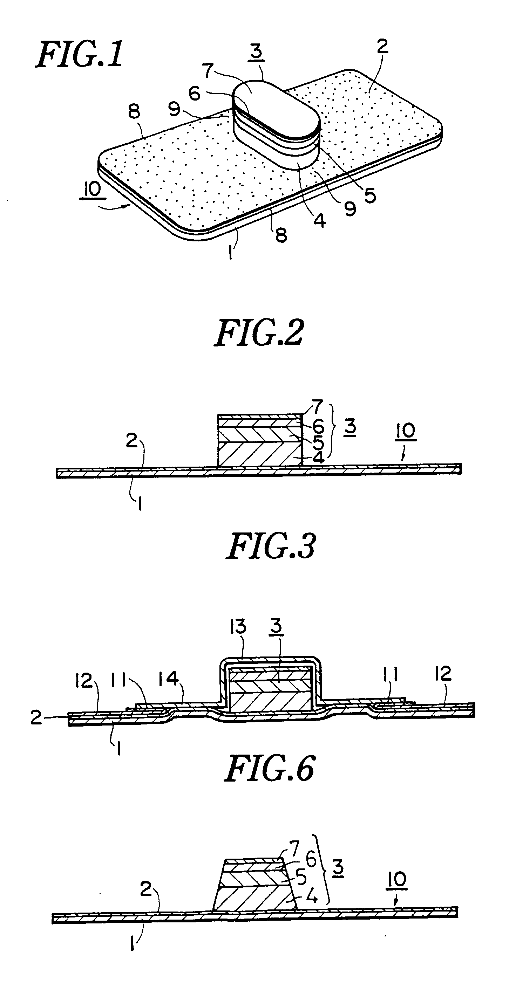

The present invention will be described hereinafter with reference to the embodiments. In Fig.1 and Fig.2, an adhesive layer 2 is formed on one surface of a base material 1. For the base material various materials such as a plastic sheet, cloth or unwoven cloth, may be employed. Here, if the ones having stretchability are employed, when the base material is stuck over the puncture area as mentioned below while stretching it, it tends to shrink after sticking, whereby the pad as mentioned below will be more effectively pressed to the puncture area.

When the sticking material is used for hemostasis of artery, the base material is preferably made of a stretchable material having a high recovery property, and the ones having a 100% extensibility, a stress exerted when extended to 100% of about 4,41 to 5,88 N/cm, and a recovery rate when extended to 100% of at least about 90% are effectively employed.

Further, the ones having a 50% extensibility, a stress exerted when extended to 50% of about 1,0 to 4,90 N/cm, and a recovery rate when extended to 50% of at least about 90% may also effectively be employed. Such a material includes, for example, fabrics having fine continuous filaments of polyurethane elastic fibers randomly laminated and bonded at the cross points.

As the adhesive of the adhesive layer 2 formed on the surface of the base material adhesives of acryl type, rubber type, silicone type, vinyl type, etc. may be appropriately employed. Particularly, when the acryl type adhesives are employed, stimulation against the skin will be reduced, such being preferable. The adhesive is provided on all or part of one surface of the base material so that it would be stuck and fixed to the wound area as mentioned below.

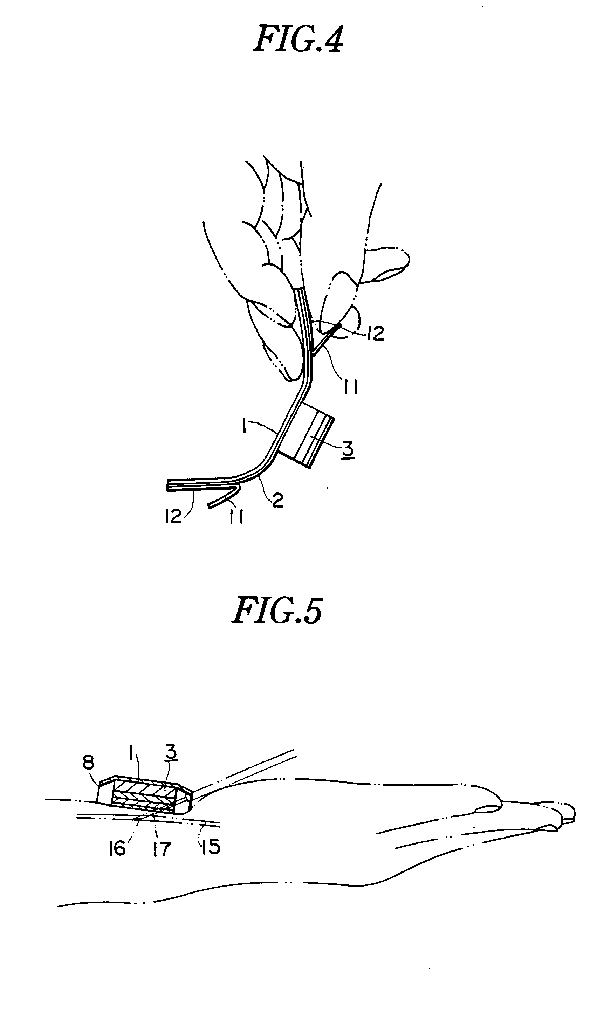

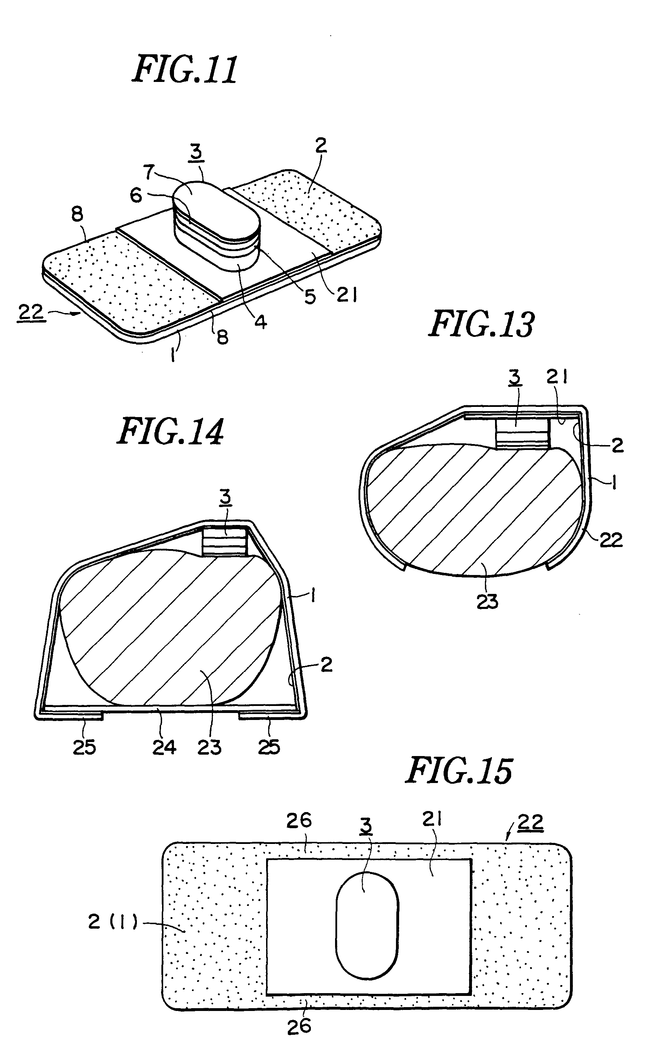

On the adhesive layer, a pad 3 is placed firmly. A pressing plate 21 of larger size than the pad 3 being inter posed between the pad and the adhesive layer of the base material 1 - see fig.11.
This pad has a size such that when the injection needle, etc. is inserted into an arterial blood vessel 15 through the skin surface of the patient, it would cover both a puncture 16 of the arterial blood vessel and a puncture 17 of the skin surface at the same time for pressing as exemplified in Fig.5.

The distance between both punctures 16 and 17 differs depending on the area of the body to be punctured, ages such as children or adult, or sex. However, the size of the pad is usually about 1 to 4 cm in the long side. The pad is formed in an appropriate shape such as rectangular, square, circle, oval, elliptical and polygon. In the case of oval, elliptical and rectangular, the ones having the ratio of the short side to the long side being up to about 1:4 are preferred. In the case of square and circle, the ratio is 1:1. Thus, the ratio is usually within the range of from about 1:1 to about 1:4.

When the pad is formed in a shape having corners, for example, rectangular, square and polygon, if the corner areas are formed in an arc shape, the stimulation against the skin surface is reduced when pressed, such being more preferable.

When the pad is formed in a elliptical shape with the long side being about 20 to 40 mm and the short side being about 10 to 20 mm, both the puncture of the artery and the puncture at the skin surface are pressed in most cases, such being preferable.

Further, this pad is preferably one having a hardness capable of securely pressing the puncture, for example, one having not only a slight elasticity but also an appropriate compression strength so that it would not be readily deformed when pressed.

This pad has such a thickness that it would press the puncture when put against the puncture area, and the thickness is at least 3 mm, and usually about 3 to 20 mm, although it differs depending on the applicable area. If the pad is too thin, pressing can not be effectively conducted.

The sticking materials having a thin pad are suitable to hemostasis for dorsalis pedis artery, etc., and the ones having a thick pad are suitable to hemostasis for femoral artery, etc. When the pad becomes thick, the pressure will be strong, but the pad may in rare cases happen to be crushed horizontally or collapsed, such being not preferable. In many cases, preferred thickness is about 5 to 13 mm.

This pad is formed in a multi-layer structure. Preferably, a cushion layer 4, a lower layer 5, an upper layer 6 and a surface layer 7 are sequentially formed from the side of the adhesive layer 2. The cushion layer 4 has an elasticity at a level of a slight hardness to obtain pressing effect, and a sufficient recovery action so that no so-called settling phenomenon will result. The lower layer 5 subsequently formed is a layer exerting the pressing action and being capable of absorbing blood, and is softer and has less nerve than the cushion layer. The upper layer 6 is softer and has less nerve than the lower layer 5, so that it would be softly in contact with the puncture and the blood would be absorbed rapidly. The surface layer 7 is formed thin, to cover hairiness, of the upper layer 6 and facilitate peeling off of the coagulated blood.

This pad is preferably made of for example the following materials. The cushion layer 4 is formed from plastic foam such as polyethylene foam having a large content of isolated cells, or compressed cotton such as compressed cotton of polyester unwoven cloth, etc. For the lower layer 5, compressed cotton such as compressed cotton of cellulose unwoven cloth, or a laminated rayon cotton, is employed. For the upper layer 6, unwoven cloth such as rayon unwoven cloth, is employed. For the surface layer, perforated polyethylene film, nylon net, rayon net, a thin cellulose unwoven cloth, etc, are employed.

An example effective for hemostasis of artery, is the one wherein the cushion layer 4 is made of polyethylene foam having a large content of isolated cells and occupies about 50 to 70 % of the entire thickness, the lower layer 5 is made of cellulose unwoven compressed cotton and occupies about 20 to 30 % of the thickness, the upper layer 6 is made of a rayon unwoven cloth and occupies about 20 % of the thickness, and the surface layer 7 is a perforated polyethylene film.

The lower layer 5 and the upper layer 6 may be formed in one layer by using a material having the properties required for both layers, to form a three-layer structure as a whole. Further, the lower layer 5, the upper layer 6 and the surface layer 7 may be formed in one layer to form a two-layer structure as a whole.

This pad is usually located at the central portion of the base material so that the sticking material can be stuck over the puncture with the adhesive layer 2 provided on the substrate 1. However, depending on the site of application, the pad may be localized at one side of the base material for easy handling.

Although this base material is sometimes formed in square, circle, oval, etc,. preferred is often a long strip shape.

When the pad is placed on the long strip-shaped base material, it is advisable to place the pad such that the long side of the pad extends in the short side direction (transverse direction) of the base material. Further, the pad covers in its long side of the puncture of arterial blood vessel and the puncture of the skin surface, whereby the pressing can readily be conducted. Thus, after that, the adhesive layer is stuck to the skin surface of the patient, whereby sure hemostasis can simply and securely be conducted. In the embodiment as shown in Fig.1 and Fig.2, an elliptical pad is placed such that the long side of the pad would extend at right angle to the longitudinal direction of the long strip-shaped base material such being preferred for pressing. However, the pad may be placed obliquely at an angle other than the right angle.

The pad is preferably placed with a space 9 of a length shorter than the thickness of the pad, i.e. a distance of usually about 3 to 10 mm, preferably about 6 mm, from each of both side edges 8, 8 of the base material. By such a structure, when this material is stuck to the skin, the portion of the space 9 of the base material is folded to cover the both sides of the pad and the pressing action is exerted inner-obliquely and downwards. Thus, the pressing action is concentrated towards the pad without scattering, whereby the thick pad is more securely prevented from moving horizontally or collapsing (Fig.5). Further, since the space 9 is narrow as mentioned above, when an injection needle is removed while pressing with the pad during the use of the sticking material, the injection needle, etc. will not be in contact with the base material having the adhesive layer, etc., and no interruption of the procedure will be caused as mentioned below.

It is advisable to cover the surface of the adhesive layer and pad of the base material of the sticking material for hemostasis 10 formed as above, with a release paper. It is preferred to pack the material with a bag for hygiene and easy handling. The one as shown in Fig.3 is obtained by covering the adhesive layer of the both sides of the base material with a release paper 12 having a folding strip 11 disposed, and further putting thereon a cap 14 made of a plastic having an enclosing portion 15 for the pad 3, whereby the pad can be hygienically maintained without distortion. Such a material may be sealed in a packaging bag obtained by weakly and removably adhering a pair of packaging sheets with a co-sealing agent, etc. or, as the case requires, may be sealed in such a packaging bag without putting on the cap (not shown in the drawing).

When this sticking material is used, referring to the ones as shown in Fig.1 to 3, the cap 14 is removed and the base material is grasped while inserting thumb between the release paper 12 and the folding strip 11 (Fig.4).

For example, when a needle of about 22 to 23 gauge (an outer diameter of 0,72 to 0,66 mm)is used for blood extraction or a catheter of about 20 gauge (an outer diameter of 0,91 mm) is used at radial artery of the wrist, before the needle or the catheter is removed, the pad is attached to the puncture area such that the long side thereof extends in the direction that the needle is inserted at the puncture area, i.e. the direction that the ductus arteriosus 15 lies, and the needle or the catheter is removed while pressing the pad. Further, one release paper is peeled off while pressing the pad, the adhesive layer is stuck to the skin such that it will lie across the direction that the ductus arteriosus lies while pulling the base material slightly, and then another release paper is picked at its folding strip and removed, and the adhesive layer is stuck to the skin while pulling the base material similarly to fix the sticking material to the skin.

By such a procedure, the pad is located over the puncture 16 of the ductus arteriosus under the skin and also covers the puncture 17 of the skin surface. Then, the pad is pressed downward by the shrink action of the stretched base material. Further, this pad presses the skin downwards strongly although the press is soft due to the multi-layer structure including the cushion layer. Thus, the area of the puncture 16 of the ductus arteriosus 15 is pressed concentratedly and strongly and closed, whereby the hemostasis can be securely conducted. By the pressing action, bleeding from the puncture 17 of the skin surface will result after removal of the needle. However, the blood will be immediately readily absorbed and held therein, whereby no blood will leak out.

After a certain period of time while this article sticks the hemostasis is complete, and the article can be removed from the skin surface, about 5-30 minutes, generally about 10 - 20 minutes after stuck to the skin surface, whereby no contamination of the clothes, etc. by the bleeding will result.

When the sticking material is adhered to the wrist as above, if the base material does not wind the entire periphery of the wrist, blood circulation of other blood vessels will not be disturbed and the hemostasis of the important area can adequately be conducted, such being advantageous.

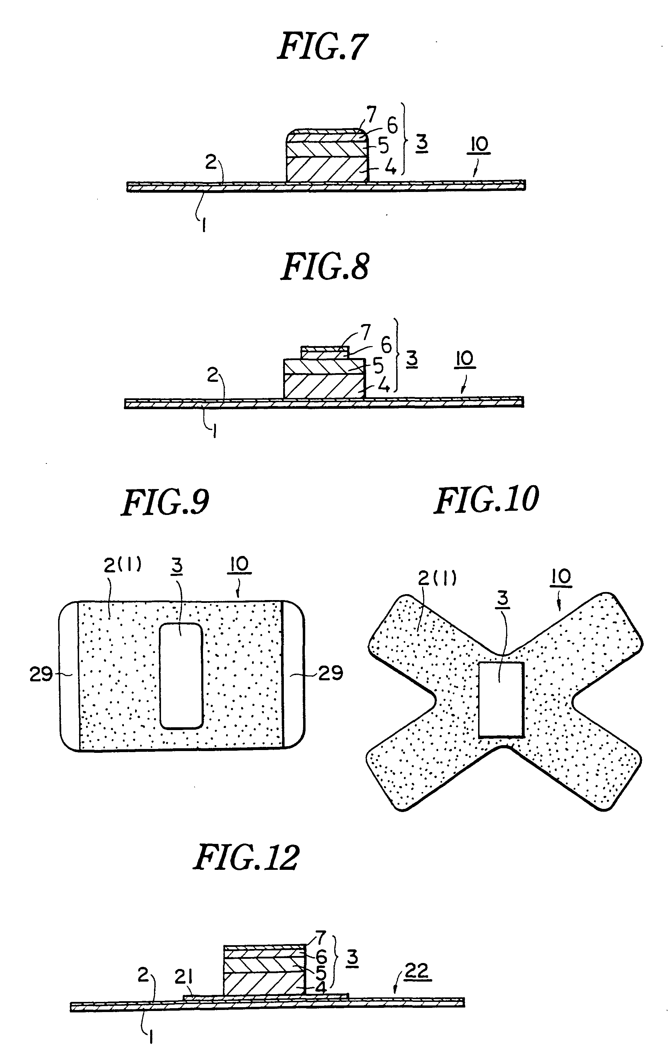

The sticking material for hemostasis as shown in Fig.6 is produced by making the shape of the pad 3 in a trapezoid shape wherein the area just above the adhesive layer of the base material is made larger than the other side. This sticking material makes it possible to press the pad against the puncture under more stable condition, whereby the pad will hardly be collapsed and the hemostasis will be effectively conducted.

In the sticking material for hemostasis as shown in Fig.7, the corners of the surface layer 7 side of the pad 3 are cut off in an arc shape so that the material can adapt to the shape of the skin surface, whereby the contact with the skin will be milder.

In the sticking material for hemostasis as shown in Fig.8, the surface layer 7 being in contact with the skin and the upper layer 6 of the pad are designed to be slightly smaller, whereby the stability when the pad is fixed is further increased and the pressing action will be made concentrated.

In the sticking material for hemostasis as shown in Fig.9, a non-adhesive portion 29 is disposed by omitting the adhesive layer 2 at both edge areas of the base material or other sticking sheet. When the sticking material is to be peeled off the skin after the hemostasis is completed, the non-adhesive portion 29 is readily grasped and thus the sticking material can readily be peeled off. The non-adhesive portion can also be disposed only at one edge area of the base material.

In the sticking material for hemostasis as shown in Fig. 10, the base material is formed in an X-shape, and a pad is placed at the central portion thereof. This base material is sometimes useful to fix and press the pad to the puncture area more securely.

The sticking material for hemostasis as shown in Figs. 6 to 10 can be stuck to the wound portion for use according to the above explanation.

Fig.11 and Fig.12 also show an embodiment. This sticking material for hemostasis has a pressing plate 21 of a thin and tough strip-shaped member made of a plastic plate, a metal plate, a plywood or the like, between the adhesive layer 2 and the pad 3. This pressing plate is formed larger in size than the pad. It is advisable to form the pressing plate to have a length of about 3 to 10 cm in the longitudinal direction of the base material and the length is usually about 4 to 7 cm. In the sticking material for hemostasis as shown in the drawings, the width of the pressing plate is made in the same size as the width of the base material and the elliptical-shaped pad is fixed on the pressing plate such that the long side of the pad would extend at the right angle to the longitudinal direction of the long strip-shaped base material. Such a structure is a preferred embodiment when the arterial blood vessel is pressed. However, the pad may be placed in an oblique direction as has been mentioned above. This pad may be placed such that it is shifted towards one side of the pressing plate.

The sticking material for hemostasis 22 can be used for hemostasis of artery in the same manner as above. The action of the stretched base material to shrink during the use, is concentrated towards the pad by the large pressing plate, and serves to strongly press it downwards. By this action, the area to which the base material is not stuck becomes wide by means'of the pressing plate, it will rarely happen to disturb the blood circulation of the ulnar artery and the vein other than the punctured radial artery of the arm portion 23. Thus, the hemostasis can be conducted by adequately pressing the puncture area without causing cyanosis, etc., and the stimulation by the adhesive against the skin will be reduced, such being preferable. Further, if the pressing plate is bent when the sticking material for hemostasis is stuck, the pressing action by means of the pad may sometimes be further strengthened by the recovery action of the pressing plate.

In the sticking material for hemostasis as shown in Fig.14 the pad 3 is placed on the adhesive layer 2 of the base material 1 as in the embodiment shown in Fig.1, and this pad is placed to cover the puncture of the blood vessel. Then, a pressing plate 24 is located on the skin surface at the opposite side of the skin surface having the pad placed, with the arterial blood vessel interposed, and both edge portions 25, 25 of the base material are stuck to the pressing plate to fix the sticking material to the wound area. This structure also provides secure hemostasis as has been made above.

In the sticking material for hemostasis as shown in Fig.15, the width of the pressing plate 21 is made narrower than the width of the base material 2. When the sticking material is stuck to the wound area, as explained with respect of Fig.5, the spacer areas 26, 26 at the both sides of the base material are folded to cover the both sides of the pressing plate, and will exert pressing action downwards and inner-obliquely. Thus, the pressing action is concentrated from the pressing plate towards the pad without scattering, thereby further preventing the thick pad from shifting horizontally of being collapsed.

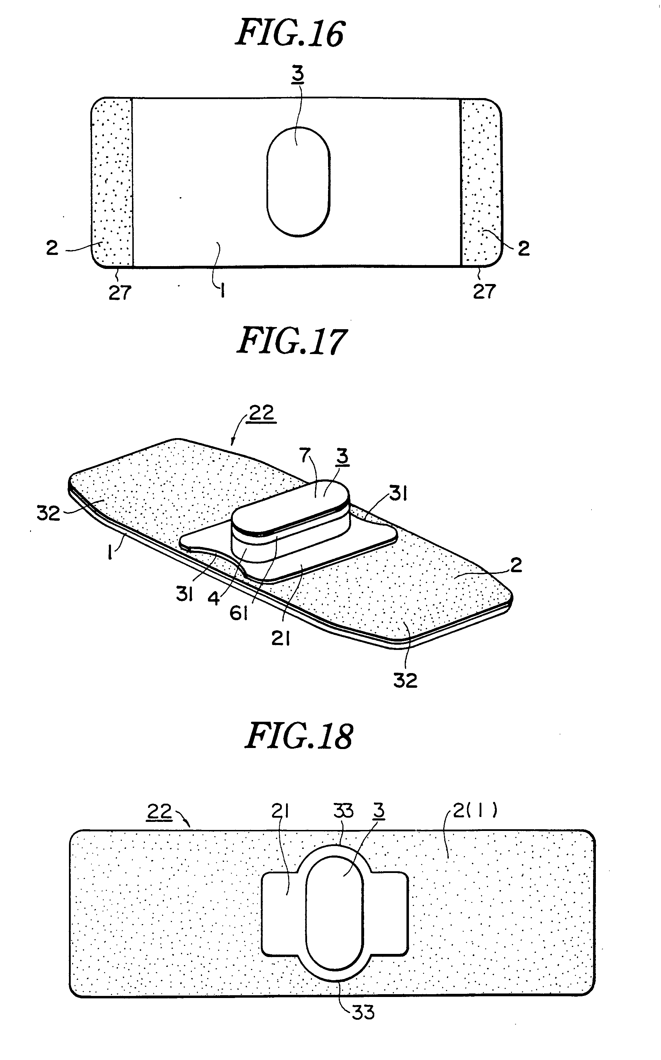

In the sticking material for hemostasis as shown in Fig.16, an adhesive layer is disposed only at both edge portions 27, 27 of the base material. This sticking material is used in the same manner as the embodiment shown in Fig.14 and, since the area of the adhesive layer is small, the pressing action and the stimulation action against the skin other than the puncture area are small, such being preferable in many cases.

In the sticking material for hemostasis as shown in Fig.17, a concave portion 31 is disposed at the central portion of the side edge of the pressing plate along the longitudinal direction of the base material, and each corner is formed in an arc shape. When this sticking material 22 is used, if the concave portions 31, 31 of the pressing plate are picked up from the base material side, the longitudinal center line of the pad will be naturally known and thus this pad can be brought in contact with both punctures securely and readily. This concave portion 31 serves to further prevent the pressing plate from being in contact with the inserted needle, etc. Since both edge portions 32 in the longitudinal direction of the base material gradually become narrower in width, the base material can readily be stretched by pulling the base material at the portions 32.

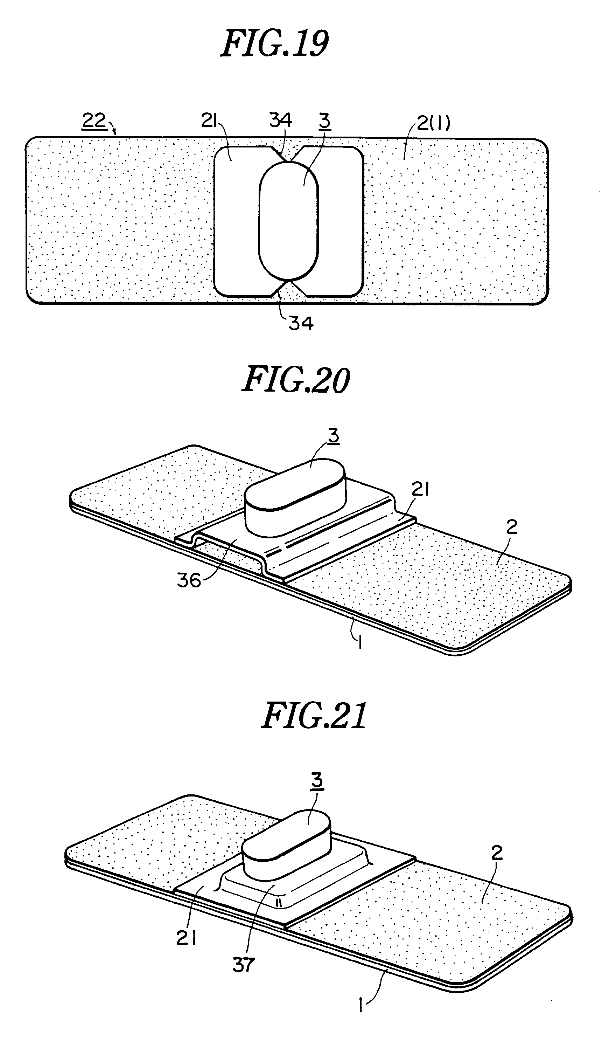

In the sticking material for hemostasis as shown in Fig.18, a convex portion 33 is disposed instead of the concave portion 31 of the pressing plate as shown in the above Fig.17. In the sticking material for hemostasis as shown in Fig.19, a V-shaped concave portion 34 is disposed. The convex portion 33 and the V-shaped concave portion 34 disposed on the pressing plate make it easy to find the position of the pad when stuck to the wound area as in the case of the concave portion 31.

Reference will be made to Figs. 20 and 21. The above-mentioned pressing plates are all explained with respect to the plate-like shape. However, the pressing plate may have a protruded portion at one part thereof and a pad may be placed on the protruded portion. In the sticking material as shown in Fig.20, a bending protruded portion 36 is formed at the central portion of the pressing plate 21. In the sticking material as shown in Fig.21, a protruded portion 37 is formed by pressing at the central portion of the pressing plate. Such a protruded portion can be formed by stacking a small strip on a large strip. In such a sticking material having a protruded portion disposed on a pressing plate, the pressing action can further be strengthened by the height of the protruded portion, whereby it is further easily applied to the hemostasis for femoral artery, etc. for which hemostasis is hardly applicable.

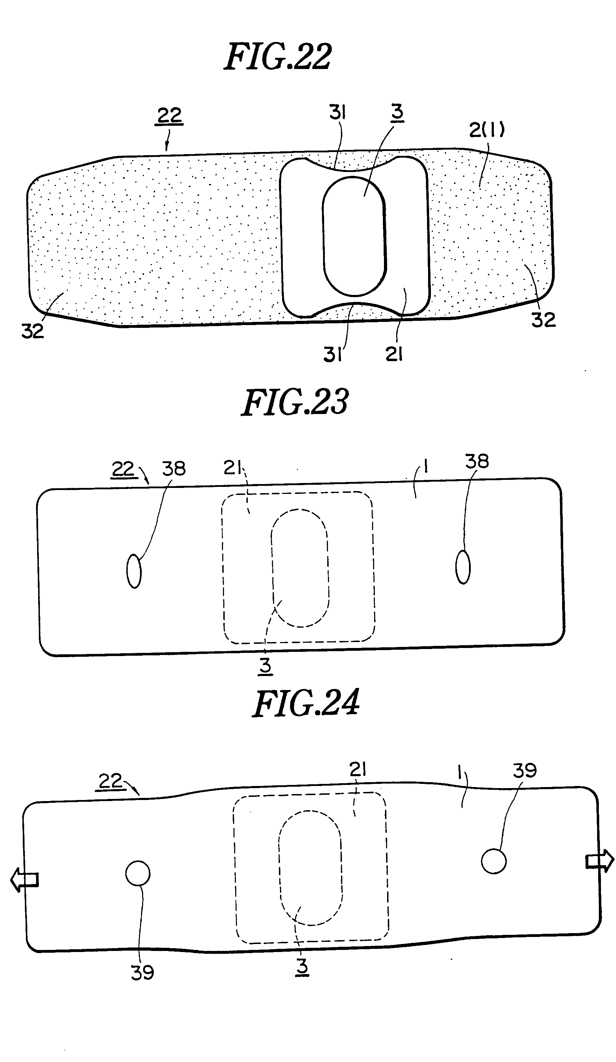

In the above mentioned embodiment, the combination of the pad and the pressing plate, is located at the central portion in the longitudinal direction of the base matérial. However, as in the sticking material for hemostasis shown in Fig.22, these pads, etc. may be placed in such a manner that these are shifted towards one side in the longitudinal direction. Such a sticking material is sometimes conveniently applicable to the ones wherein the blood vessel such as radial artery, ulnar artery of dorsalid pedis artery, is locally placed on the arm, etc.

Reference will be made to Figs. 23 and 24. In Fig.23, distorted circular figures 38 are indicated at the surface of a stretchable subbase material opposite to the adhesive layer. When the sticking material is used, by pulling the base material, the base material expands and the distorted circular figures 38 will become circle figures 39 (Fig.24). When the base material is stuck to the skin surface under this condition, the desired pressing action can readily be obtained by the shrinking base material. As such an index showing the degree of extension of the base material, various indications such as an equilateral triangle can be used in addition to the above circle figure.

The bleeding stoppage to the ductus arteriosus is mentioned above. The blood extraction, dip of water, blood transfusion, artifical dialysis, etc. are carried out by puncturing the injector needle, catheter, medical tube, etc. into the venous blood through the surface of skin.

After pulling the injector needle out, the venipunctured portion also starts bleeding, and it is therefore necessary to stop bleeding by pressing the absorbent cotton or gauze by finger strongly and pressing the absorbent cotton by adhesive tapes and/or flexible bandage, etc. However, it takes about ten minutes and more to stop bleeding by pressing the punctured portion by finger or other means fixedly, which is not only troublesome but also unfavourable, because omission of such a steady press leads to hypodermic bleeding and the blood which is blurred over from the aperture of punctured portion is likely to spoil clothings of the patient. Some patients are not in the position to press the punctured portion by fingers of other means.

Furthermore, in case of a thin injector needle, the aperture of the punctured portion of the blood vessel is also small, and the bleeding stoppage is relatively easy to the venous blood. On the other hand, in case of a thick injector needle, the aperture of the punctured portion becomes large, which results in bleeding of a large quantity of blood, and the bleeding stoppage is more difficult than the one in case of the thin injector needle. The present invention can stop bleeding of artery. For example, when the relatively thick injector needle of 16 to 18 gauge (outer diameter 1,67 to 1,28 mm) is used for blood extraction and blood transfusion, sure bleeding stoppage is obtained. Of course, the bleeding stoppage is carried out more easily in case of using a thin injector needle.

### Measurement of the pressing force

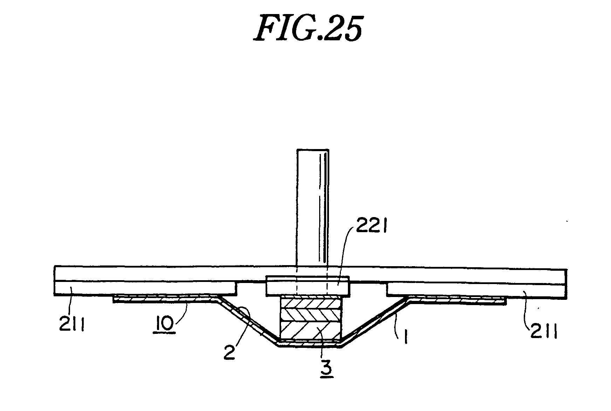

The adhesive layer of the product was stuck to a platform for measurement 211 made of Bakelite as shown in Fig.25, the pad is pressed with a plate 221 of 20 mm diameter, so that the surface of the plate would be adjusted to the surface of the platform for measurement with a rheometer, and the pressing force after 1 minute was measured. The measurement was conducted with respect to 10 samples.
Results: The pressing force was 35000±1200 N/m² (360±12 p/cm²)

### Consideration

Accordingly, sufficient pressing force was obtained by the product and this product was confirmed to be useful as a hemostatic material for artery.

### EXAMPLE 1

Product 1 was prepared and the results of measurement of the force of pressure, pressure sense numbness and change of palm color when this product is used, are shown below.

### Product 1

Base material: obtained by randomly laminating fine continuous filaments comprising 100 % of polyurethane elastic fibers, and bonding the crossing points of respective filaments.

The properties are shown below:

| | |
|---|---|
| Stress at 50 % extension | Longitudinal direction 2,65 N/cm |
| | Latitudinal direction 2,74 N/cm |
| Recovery at 50 % extension | Longitudinal direction 90 % |
| | Latitudinal direction 90 % |
| Breaking strength | Longitudinal direction 12,7 N/cm |
| | Latitudinal direction 9,8 N/cm |

The above material was cut in a rectangular shape of 45 x 100 mm having arc-shaped corners, the latitudinal direction of which being a long side.
Adhesive layer: Acryl type adhesive as coated to 38 + 2 g/m²
Pressing plate: A poyvinylchloride plate having a thickness of 1 mm was cut in a size of 45 x 50 mm and stuck to the adhesive layer at the central portion of the sticking material, while adjusting the edges.
Pad: The surface layer is cellulose unwoven cloth having a thickness of 0.05 mm.

The upper layer is rayon unwoven cloth having a thickness of 2 mm

The lower layer is a compressed cotton of cellulose unwoven cloth having a thickness of 2 mm.

The cushion layer is a sheet of a foam of polyethylene isolated cells having a thickness of 5 mm.

Shape: An elliptical shape having a long side of 27 mm, a short side of 15 mm and a thickness of 9 mm.

This pad is fixed at the central portion of the pressing plate such that the long side of the pad extends in the transverse direction of the base material.

### Position, time and method for sticking

The pad was put on the radial artery of left wrist while extending the sticking material to 140 mm, and then the sticking material was stuck in the same manner as in Fig.13 for 1 minute.

### EXAMPLE 2

### Product 2

Product 2 was prepared by using the same base material, adhesive and pad as product 1 provided that the pad was placed at the central portion of the base material.
Pressing plate: A polyvinylchloride plate with a thickness of 1,1 mm was cut in a size of 45 x 70 mm.

### Position, time and method for sticking

The pad was put on the radial artery of left wrist and the pressing plate was located at the side opposite to the pad. Then, while extending the base material to 140 mm, it was stuck to the pressing plate in the same manner as in Fig.14 for 1 minute.

### Contrast Example compared with Examples 1 and 2

There is not a pressure plate in this Contrast example. With other points, the same as Example 1.

### Items of measurement and observation

Measurement of the force of pressure under the pad portion:

At the carpal portion of radial artery, a pouch of a catheter for measurement of cerebrospinal pressure, connected-to a piezo-electric transducer, was non-invatively fixed. The sticking material was stuck so that the pad portion is located on the pouch. Then, the pressing force at the pad portion was measured.

The pressure sense, numbness, change of palm color as a side effect:

1 minute after the sticking,the testee was asked to express verbally the pressing feeling and the numbness, and the discoloration of palm was observed with naked eyes.

Testees: six persons

### Method ef evaluation

The force of pressure at the pressed area was read from the recording sheet and then converted to numerals (mm Hg). The average number thereof was determined.

The pressure sense, numbness and change of palm color were evaluated by four steps of "non, weak, medium and strong". These steps were called 0, 1, 2 and 3 respectively, and the average number thereof was determined.

### Results

| | Example 1 | Example 2 | Contrast |
|---|---|---|---|
| Force of pressure (average, mm Hg) | 246 | 143 | 85 |
| Pressure sense (average) | 3,0 | 2,2 | 1,5 |
| Numbness (average) | 1,2 | 0,8 | 1,0 |
| Change of palm color (average) | 0,7 | 0,5 | 1,2 |

### Consideration

In each of examples 1 and 2, larger pressing force was obtained as compared with the contrast example, and thereby the pressing feeling is slightly strong. However, the change of palm color is less than the control and thus improved. Accordingly, the examples 1 and 2 hardly influence human bodies, and are effective and useful to hemostasis of artery.

### EXAMPLE 3

Product 3 was prepared and the results of measurement of the pressing force, side effect (pressing feeling, numbness, discoloration of palm), the time required for disappearance of the pressure marks, and stimulus of the skin surface by changing the extension length of the base material variously are shown below:

### Product 3

The whole structure is shown in Fig.17.
Base material: The base material is obtained by randomly laminating fine continuous filaments comprising 100 % of polyurethane elastic fibers, and bonding the crossing points of respective filaments. The properties are shown as below:

| | |
|---|---|
| Weight | 100 g/m² |
| Stress at 50 % extension | Longitudinal direction 4,9 N/cm/20 mm |
| | Latitudinal direction 3,63 N/cm/20 mm |
| Recovery at 100 % extension | Longitudinal direction 87 % |
| | Latitudinal direction 87 % |
| Strength of extension | Longitudinal direction 15,7 N/cm/20 mm |
| | Latitudinal direction 11,76 N/cm/20 mm |
| Extension | Longitudinal direction 370 % |
| | Latitudinal direction 380 % |

The above base material was cut in a rectangular shape of 40 mm x 126 mm having gradually narrowed edges, the latitudinal direction being a long side (Fig.17).

### Adhesive layer: Acryl type adhesive was coated to 40 g/m².

The properties of adhesive coated on the above base, is as follows:

| | |
|---|---|
| Adhesive strength | 1,96 N/cm/15mm |
| Tack (rolling ball method) | No. 31 |
| Pressing plate | Polypropylene plate of 1 mm thickness was cut in a square of 36 mm x 36 mm with arc-shaped corners, having a convex on an opposite side (Fig.17). |

This pressing plate is adhered to the adhesive layer at the central portion of the sticking material such that the above mentioned convex is along with the longitudinal direction of the base material.
Pad: The surface layer is cellulose unwoven cloth having a thickness of 0,05 mm.

The upper layer is rayon unwoven cloth having a thickness of 3mm.

The cushion layer is a sheet of a foam of polyethylene isolated cell having a thickness of 6 mm.

The property of this sheet of foam is as follows:

| | |
|---|---|
| density at glande | 0,067 g/cm³ |
| compressed hardness | 0,64 KG/cm² |
| shape | an elliptical shape having a long side of 27 mm, a short side of 15 mm and a thickness of 9 mm. |

This pad is fixed at the central portion of the pressing plate such that the long side of the pad extends in the transverse direction of the base material (Fig.17).

### Position, time and method for sticking

The pads were put on the radial arteries of left and right wrists, and the sticking material was stuck for a minute under the following four conditions:
(1) under non-extension of the sticking material
(2) while extending the sticking material by 1 cm each side (total 2 cm)
(3) while extending the sticking material by 2 cm each side (total 4 cm)
(4) while extending the sticking material by 3 cm each side (total 6 cm)

Thus, the pressure force of this sticking material and the condition of the skin after applying this sticking material is known.The duration of application was one hour (1 hr).

### Testing method

Measurement of force of pressure under the pad portion:
The same as in EXAMPLE 1 and 2. The pressure force of the pressure body when adhering and one hour later are measured from the record papers and are indicated as numbers (mm Hg) whose means ± standard deviation are indicated.
   Side effect (pressure sense, change of palm color and numbness): One hour after adhesion, pressure sense and numbness of the testees are inquired, and change of palm color was observed by eyes.
The evaluations (rating) are as follows:
   five stages: none, weak, medium, rather strong and strong; each step is referred to 0, 1, 2, 3 and 4, whose average means number ± standard deviation is determined.

Adhesion and peeling condition: The condition of adhesion, and the residue of the adhesive is evaluated in peeling the adhesive bandage one hour later.
Time required for disappearance of the pad mark: After peeling the sticking material, pressure marks by the pad are observed by eyes and the time for disappearance of the pad marks is measured.
Dermal irritation index: The reaction of the skin with the adhesive attached was evaluated according to the following standard: four hours later and 24 hours later after peeling, respectively, and the dermal irritation index was determined.

| | | |
|---|---|---|
| - | 0 | no reaction |
| ± | 0,5 | weak erythema |
| + | 1,0 | erythema |
| ++ | 2,0 | erythema + edema |
| +++ | 3,0 | erythea + edema + palule ore serous palule or vesicle |
| ++++ | 4,0 | bulla |

Dermal irritation index = total/testees x 100
Sample number of each extension: 12

### Results

(1) adhesion and peeling condition: No peeling (removal) is found, and favourable adhesion condition is kept. No residue of the adhesive is found at the skin after peeling.
(2) The pressure force, side effect (pressure sense, change of palm color, numbness), time required for disappearance of the pressure marks, skin stimulus indication number are as follows:

| The force of pressure (mmHg) | | | | |
|---|---|---|---|---|
| | 0 cm | 1 cm | 2 cm | 3 cm |
| Initial | 11±6 | 79±18 | 112±19 | 141±26 |
| 1 hour after application | 9±6 | 68±16 | 98±17 | 121±17 |

| Side effect (5 classes) | | | | |
|---|---|---|---|---|
| | 0 cm | 1 cm | 2 cm | 3 cm |
| Pressure sense | 0,0±0,0 | 0,4±0,6 | 0,9±0,6 | 1.4±0,6 |
| Change of palm color | 0,0±0,0 | 0,2±0,4 | 0,3±0,4 | 0,5±0,5 |
| numbness | 0,0±0,0 | 0,2±0,4 | 0,2±0,4 | 0,5±0,6 |

| Disappearance of the pad mark (hr) | | | | |
|---|---|---|---|---|
| | 0 cm | 1 cm | 2 cm | 3 cm' |
| disappearance time | 0,8±0,4 | 2,7±0,5 | 3,2±0,6 | 3,6±0,6 |

| Dermal irritation index (D.I.I.) | | | | |
|---|---|---|---|---|
| | 0 cm | 1 cm | 2 cm | 3 cm |
| 4 hr | 0,0 | 0,0 | 0,0 | 4,2 |
| 24 hr | 0,0 | 0,0 | 0,0 | 0,0 |

### Consideration

As a whole, the adhering condition is favourable, since no peeling is found, and no residue of the adhesive is found after peeling.

When the base material is extended by 2 cm and 3 cm respectively, the pressure force over the usual systolic blood pressure is attained. One hour after sticking, the pressure force decreased by 10 - 20 mmHg, but it is favourable in view of the release of the pressure after hemostasis.

The side effect after one hour usage of the product 3 is very small, i.e., there is no problem in use.

When the base material is extended by 3 cm, the base material surrounds the periphery of the wrist, but no side effect is found. This is because the pressing plate allows the ulnar artery's blood to flow freely.

Thus, when used by extending about 2 cm, the product 3 can give the proper pressure action, and various side effects are very small even after the long use. Therefore, it serves as the sticking product of hemostasis of artery.

According to the present invention, as mentioned above, it is possible to securely conduct the hemostasis of the puncture of artery, and effective hemostasis can be made to arteries located close to bones such as ulnar artery and dorsalis pedis artery in addition to radial artery. Further, the present invention is applicable to femoral artery.
Effective hemostasis can be made to a vein also as has been applied to the artery. Further, the procedure of use is easy and the structure of the sticking material is simple, whereby the production can be made economically.

## Claims

1. A sticking compressing material for hemostasis comprising an elastically stretchable and long strip-shaped base material (1) having a recovering property with an adhesive layer (2) disposed on one surface thereof and carrying a pad (3) of suitable form, dimension and rigidity for exerting a compressing action when applied to the point or region of hemorrhage, so as to ensure complete flattening and closing of a vein when applied against a puncture area of said vein, **characterised in that** for hemostasis of a blood vessel being a vein or artery, the body is a pressing plate (21) of larger size than the pad (3) and is interposed between the pad (3) and the adhesive layer (2) on the base material (1) and that the pad (3) has a multi-layer-structure comprising, on said base material (1) in a sequence, a cushion layer (4) having a high pressing effect in a thickness direction, a lower layer (5) which absorbs blood and has a pressing effect, an upper layer (6) which rapidly absorbs blood, and a surface layer (7) which prevents hairiness of the upper layer (6) and deposition of coagulated blood.

2. A sticking compressing material for hemostasis according to claim 1 wherein the pad (3) has an elliptical shape having a long side of from 20 to 40 mm, a short side of from 10 to 20 mm and a thickness of from 8 to 12 mm, and the pad is placed such that its long side extends in the transverse direction of the base material (1).

3. A sticking compressing material for hemostasis according to claims 1 or 2, wherein the pad (3) has a ratio of short side to long side of from 1:1 to 1:4.

4. A sticking compressing material for hemostasis according to any of claims 1 to 3, **characterised in that** each of the side edges of the pressing plates (21) extending in the longitudinal direction of the base material (1) is provided with a concave portion (31).

5. A sticking compressing material for hemostasis according to any of claims 1 to 3, **characterised in that** each of the side edges of the pressing plates (21) extending in the longitudinal direction of the base material (1) is provided with a convex portion (33).

## Patentansprüche

1. Klebendes Kompressenmaterial zur Blutstillung mit einem elastisch dehnbaren und langen streifenförmigen Trägermaterial (1) mit Rückstellfähigkeit das auf einer Fläche eine Klebschicht (2) aufweist und ein Kissen (3) von geeigneter Form, Abmessung und Steifheit trägt, das beim Auflegen auf den Punkt oder Bereich der Blutung eine Kompressionswirkung ausübt, um ein völliges Flachdrücken und Verschließen einer Vene zu gewährleisten, wenn es gegen einen Punktionsbereich der Vene angelegt wird, **dadurch gekennzeichnet**, daß zur Blutstillung eines Blutgefäßes, das eine Vene oder Arterie ist, der Körper eine Druckplatte (21) ist, die größer als das Kissen (3) und zwischen den Kissen (3) und der Klebschicht (2) auf dem Trägermaterial (1) angeordnet ist und daß das Kissen (3) eine Mehrschichtenstruktur hat, die auf dem Trägermaterial (1) der Reihe nach eine Polsterschicht (4) mit hoher Druckwirkung in einer Dickenrichtung, eine untere Schicht (5), die Blut absorbiert und eine Druckwirkung hat, eine obere Schicht (6), die rasch Blut absorbiert, und eine Oberflächenschicht (7), die Haarigkeit der oberen Schicht (6) und Ablagerung von koaguliertem Blut verhindert, aufweist.

2. Klebendes Kompressenmaterial zur Blutstillung nach Anspruch 1, wobei das Kissen (3) eine elliptische Form mit einer langen Seite von 20 bis 40 mm, einer kurzen Seite von 10 bis 20 mm und eine Dicke von 8 bis 12 mm hat und so angeordnet ist, daß seine lange Seite sich in der Querrichtung des Trägermaterials (1) erstreckt.

3. Klebendes Kompressenmaterial zur Blutstillung nach Anspruch 1 oder 2, wobei das Kissen (3) ein Verhältnis kurze Seite zu langer Seite von 1:1 bis 1:4 hat.

4. Klebendes Kompressenmaterial zur Blutstillung nach einem der Ansprüche 1 bis 3, **dadurch gekennzeichnet**, daß jede der Seitenkanten der Druckplatten (21), die sich in der Längsrichtung des Trägermaterials (1) erstrecken, mit einem konkaven Abschnitt (31) ausgebildet ist.

5. Klebendes Kompressenmaterial zur Blutstillung nach einem der Ansprüche 1 bis 3, **dadurch gekennzeichnet,** daß jede der Seitenkanten der Druckplatten (21), die sich in der Längsrichtung des Trägermaterials (1) erstrecken, mit einem konvexen Abschnitt (33) ausgebildet ist.

## Revendications

1. Pansement compresseur pour hémostase comprenant un matériau de base en forme de longue bande élastique étirable (1) ayant des propriétés de recouvrement avec une couche adhésive (2) disposée sur l'une des surfaces de ce dernier et portant un coussinet (3) de forme, dimension et rigidité appropriés, pour exercer une action de compression quand appliqué sur le point ou la région de l'hémorragie, de manière a assurer un aplatissement complet et une suture de la veine quand appliqué contre la partie piquée de la dite veine, **caractérisé en ce que** pour l'hémostase d'un vaisseau sanguin que ce soit une veine ou une artère, le corps est une plaque de pression (21) de taille plus large que le coussinet (3) et est interposée entre le coussinet (3) et la couche adhésive (2) du matériau de base (1) et que ce coussinet (3) a une structure multicouches comprenant, sur le dit matériau de base (1) par séquence, une couche d'amortissement (4) ayant un haut effet de pression en direction de l'épaisseur, une couche inférieure (5) qui absorbe le sang et a un effet de pression, une couche supérieure (6) qui absorbe rapidement le sang, et une couche de surface (7) qui prévient l'abondance des poils de la couche supérieure (6) et le dépôt de sang coagulé.

2. Pansement compresseur pour hémostase conformément à la revendication 1 dans lequel le coussinet (3) a une forme elliptique ayant un côté long de 20 mm à 40 mm, un côté court de 10 mm à 20 mm et une épaisseur de 8 à 12 mm, et le coussinet est placé de telle manière que le côté long s'étend en direction transversale du matériau de base.

3. Pansement compresseur pour hémostase conformément aux revendications 1 et 2, dans le quel le coussinet (3) à un rapport entre le côté court et le côté long de 1 :1 à 1 :4.

4. Pansement compresseur pour hémostase conformément avec l'une quelconque des revendications 1 à 3, **caractérisé en ce que** chaque côté des bords des plaques de pression (21) s'étendant en direction longitudinale du matériau de base (1) sont pourvus d'une partie concave (31).

5. Pansement compresseur pour hémostase conformément avec l'une quelconque des revendications 1 à 3, **caractérisé en ce que** chaque côté des bords des plaques de pression (21) s'étendant en direction longitudinale du matériau de base (1) sont pourvus d'une partie convexe (33).
